# EUROPEAN PATENT SPECIFICATION

(11) **EP 4 547 109 B1**
(45) Date of publication and mention of the grant of the patent: **16.09.2026**
(21) Application number: 23845740.2
(22) Date of filing: 31.07.2023
(51) Int. Cl.: A61B 6/00, A61B 6/03, A61B 6/04, A61B 5/055, A61B 5/00

(54) **METHODS AND SYSTEMS FOR IMAGING**
VERFAHREN UND SYSTEME ZUR BILDGEBUNG
PROCÉDÉS ET SYSTÈMES D'IMAGERIE

(30) Priority: 29.07.2022 CN 202210907320
(43) Date of publication of application: 07.05.2025
(73) Proprietor: Shanghai United Imaging Healthcare Co., Ltd., Shanghai 201807 (CN)
(72) Inventor: XU, Xiaochun, Shanghai 201807 (CN); YE, Qing, Shanghai 201807 (CN)
(74) Representative: Wang, Bo
(86) International application number: PCT/CN2023/110448
(87) International publication number: WO 2024/022536

(56) References cited:
- EP-A1- 3 396 630
- WO-A1-2020/028607
- CN-A- 107 095 691
- CN-A- 110 755 100
- CN-A- 111 462 885
- US-A1- 2017 103 551
- US-A1- 2019 059 831
- US-A1- 2020 170 605

## Description

### CROSS-REFERENCE RELATED TO APPLICATIONS

The present disclosure claims priority of Chinese Patent Application No.202210907320.X, filed on July 29, 2022.

### TECHNICAL FIELD

The present disclosure relates to the field of medical technology, and in particular to methods and systems for imaging.

### BACKGROUND

The Positron Emission Tomography (PET) parameter imaging function and its corresponding applications are still at an early stage of development. US 2017/103551 A1 discloses a method for reconstructing a PET multi-bed image, which may be applied to a PET scan having an axial overlapping region existed between every two adjacent beds. One of the major needs of parameter imaging is to obtain information about the three-dimensional distribution of drugs and nuclides over time. However, given the low drug dose of PET imaging and the limited amount of nuclide decay information, it is inherently difficult to achieve dynamic imaging with less count information in a short time. In addition, PET parameter imaging also requires a continuous input function as the basis to obtain comprehensive coverage of body-wide information including the blood pool region for input function and lesion area.

Therefore, a method for determining a continuous input function is urgently needed.

### SUMMARY

The invention is set out in the appended set of claims.

### BRIEF DESCRIPTION OF THE DRAWINGS

The present disclosure is further descritable position in terms of exemplary embodiments. The exemplary embodiments are descritable position in detail with reference to the drawings. The embodiments are non-limiting exemplary embodiments, in which like reference numerals represent similar structures throughout the several views of the drawings, and wherein:
FIG. 1 is a schematic diagram illustrating an exemplary system for imaging according to some embodiments of the present disclosure;
FIG. 2 is a schematic diagram illustrating an exemplary system for imaging according to some embodiments of the present disclosure;
FIG. 3 is a flowchart illustrating an exemplary process for imaging according to some embodiments of the present disclosure;
FIG. 4 is a schematic diagram illustrating a process for imaging according to some embodiments of the present disclosure;
FIG. 5 is a schematic diagram illustrating an input function according to some embodiments of the present disclosure;
FIG. 6 is a flowchart illustrating an exemplary process for imaging according to some embodiments of the present disclosure;
FIG. 7 is an exemplary schematic diagram illustrating an exemplary machine learning model according to some embodiments of the present disclosure.

### DETAILED DESCRIPTION

To more clearly illustrate the technical solutions of the embodiments of the present disclosure, the accompanying drawings that need to be used in the description of the embodiments would be briefly introduced below. Obviously, the accompanying drawings in the following description are merely some examples or embodiments of the present disclosure, and those skilled in the art may apply the present disclosure to other similar situations according to the drawings without any creative effort. Unless obviously obtained from the context or the context illustrates otherwise, the same numeral in the drawings indicates the same structure or operation.

It will be understood that the terms "system," "device," "unit," and/or "module" used herein are used to distinguish different components, elements, parts, input function segments, or assemblies of different levels. However, the terms may be displaced by other expressions if they may achieve the same purpose.

As used in the present disclosure and the appended claims, the singular forms "a," "an," and "the" are intended to include plural referents, unless the content clearly dictates otherwise. Generally, the terms "comprise" and "include" only imply that the clearly identified steps and elements are included, but these steps and elements may not constitute an exclusive list, and the method or device may further include other steps or elements.

The flowcharts used in the present disclosure illustrate operations that systems implement according to some embodiments of the present disclosure. It is to be expressly understood, the operations of the flowcharts may be implemented not in order. Conversely, the operations may be implemented in an inverted order, or simultaneously. Moreover, one or more other operations may be added to the flowcharts. One or more operations may be removed from the flowcharts.

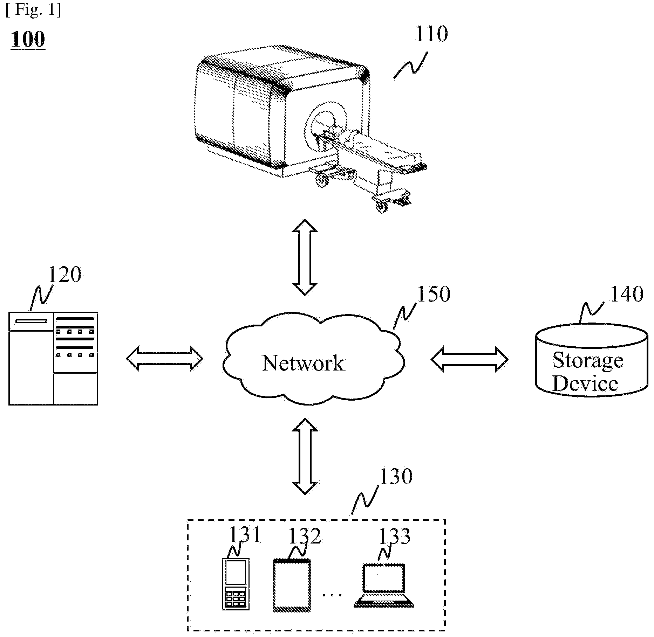

FIG. 1 is a schematic diagram illustrating an exemplary system for imaging according to some embodiments of the present disclosure. In some embodiments, as shown in FIG. 1, an application scenario 100 of the system for imaging may include at least an imaging device 110, a processing device 120, a terminal device 130, a storage device 140, and a network 150.

The imaging device 110 may scan an object within a detection area or a scanning area to obtain scanning data for that object. In some embodiments, the object may include a biological object and/or a non-biological object. For example, the object may include a patient, an artificial object, etc. In some embodiments, the object may include a specific portion of the body, such as the head, the chest, the abdomen, etc., or any combination thereof. In some embodiments, the object may include a specific organ, such as the heart, the esophagus, the trachea, the bronchus, the stomach, the gallbladder, the small intestine, the colon, the bladder, the ureter, the uterus, the oviduct, etc., or any combination thereof. In some embodiments, the object may include a region of interest (ROI), such as a tumor, a node, etc.

In some embodiments, the imaging device 110 may be or include a Positron Emission Tomography (also referred to as PET) scanner. In some embodiments, the imaging device 110 may include a single-modality scanner and/or a multi-modality scanner. The multi-modality scanner may include a PET-CT scanner, a PET-MRI imaging device, etc., or any combination thereof. The above descriptions of the imaging devices are merely provided for the purpose of description only, which are not intended to limit the scope of the present disclosure.

The processing device 120 may process data and/or information obtained from the imaging device 110, the terminal device 130, the storage device 140, and/or other components of application scenario 100 of the system for imaging. For example, the processing device 120 may obtain image data from the imaging device 110, the terminal device 130, the storage device 140, and analyze and process the obtained image data. As another example, the processing device 120 may obtain a plurality of local input functions of an object. Each of the plurality of local input functions may correspond to or be of a scanning area. A local input function may be acquired based on a scan of one of a plurality of table positions. Adjacent table positions in the plurality of table positions may have an overlapping scanning area. The processing device 120 may also obtain a local input functions of at least two overlapping scanning areas; and determine a continuous input function of the target scanning area based on the local input functions of the at least two overlapping scanning areas.

In some embodiments, the processing device 120 may be a single server or a group of servers. The server group may be centralized or distributed. In some embodiments, processing device 120 may be local or remote. For example, the processing device 120 may access the information and/or data from imaging device 110, terminal device 130, and/or storage device 140 through the network 150. As another example, the processing device 120 may be directly connected to the imaging device 110, the terminal device 130, and/or the storage device 140 to access the information and/or data. In some embodiments, the processing device 120 may be realized on a cloud platform. For example, the cloud platform may include a private cloud, a public cloud, a hybrid cloud, a community cloud, a distributed cloud, an inter-cloud, a multi-cloud, etc., or any combination thereof.

In some embodiments, the processing device 120 and the imaging device 110 may be integrated into one device. In some embodiments, the processing device 120 and the imaging device 110 may be directly or indirectly connected to act jointly to realize the processes and/or functions of the present disclosure.

In some embodiments, the processing device 120 may include an input device and/or an output device. The input device and/or output device realize an interaction with a user (e.g., setting a scanning parameter, etc.). In some embodiments, the input device and/or the output device may include a display, a keyboard, a mouse, a microphone, etc., or any combination thereof.

The terminal device 130 may be in communication and/or connection with imaging device 110, processing device 120, and/or storage device 140. In some embodiments, the interaction with the user may be achieved through the terminal device 130. In some embodiments, the terminal device 130 may include a mobile device 131, a table positiont 132, a laptop 133, etc., or any combination thereof. In some embodiments, the terminal device 130 (or all or some of its functions) may be integrated in the processing device 120.

The storage device 140 may store data, instructions, and/or any other information. In some embodiments, the storage device 140 may store the data and/or information (e.g., scanning parameters, image data, input functions, etc.) obtained from the imaging device 110, the processing device 120, the terminal device 130. In some embodiments, the storage device 140 may store data and/or instructions that processing device 120 uses to perform or use to accomplish the exemplary process descritable position in the present disclosure.

In some embodiments, the storage device 140 may include one or more storage components, each of which may be a separate device or may be a portion of another device. In some embodiments, the storage device 140 may include a random-access memory (RAM), a read-only memory (ROM), a mass storage, a removable memory, a volatile read-write memory, etc., or any combination thereof. In some embodiments, the storage device 140 may be implemented on the cloud platform. In some embodiments, the storage device 140 may be a portion of imaging device 110, the processing device 120, and/or the terminal device 130.

The network 150 may include any suitable position network capable of facilitating the exchange of information and/or data. In some embodiments, at least one component of the application scenario 100 of the system for imaging (e.g., imaging device 110, processing device 120, terminal device 130, storage device 140) may exchange the information and/or data with at least one other component of the application scenario 100 of the system for imaging through network 150. For example, the processing device 120 may obtain the image data from the imaging device 110 through the network 150, etc.

It should be noted that the above descriptions of the application scenario 100 of the system for imaging are merely provided for the purpose of description, which are not intended to limit the scope of the present disclosure. For those skilled in the art, a variety of amendments or variations may be made based on the description of the present disclosure. For example, the application scenario 100 of the system for imaging may perform similar or different functions on other devices. However, these variations and amendments do not depart from the scope of the present disclosure.

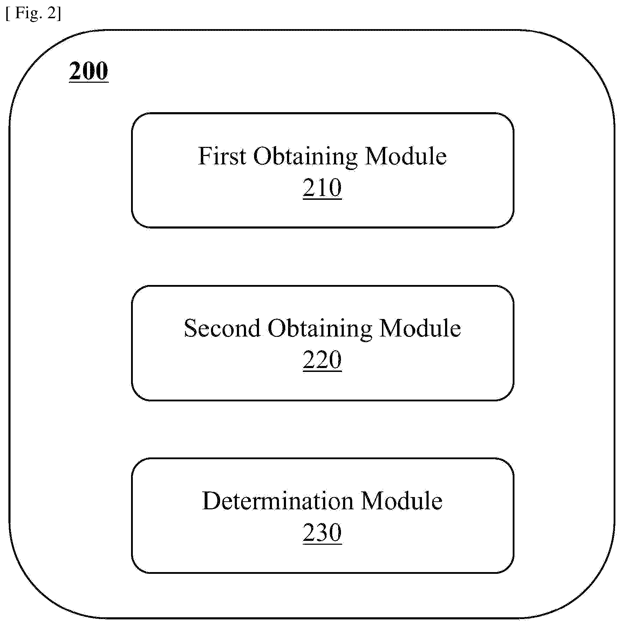

FIG. 2 is a schematic diagram illustrating a module of an exemplary system for imaging according to some embodiments of the present disclosure. As shown in FIG. 2, in some embodiments, a system for imaging 200 may include a first obtaining module 210, a second obtaining module 220, and a determination module 230. In some embodiments, functions corresponding to the system for imaging 200 may be performed by the processing device 120.

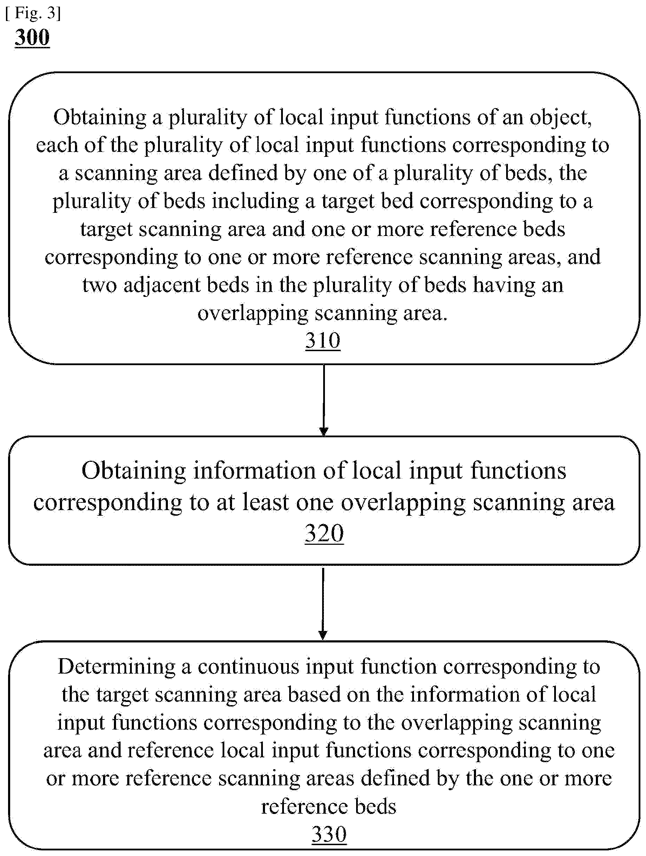

The first obtaining module 210 may be configured to obtain a plurality of local input functions of an object, each of the plurality of local input functions being of a scanning area of the object, one of the plurality of local input functions being obtained based on a scan for one of a plurality of table positions where a table for placing the object is located, wherein adjacent table positions in the plurality of table positions correspond to an overlapping scanning area. More descriptions of obtaining a plurality of local input functions may be found in operation 310 of FIG. 3 and related descriptions thereof.

The second obtaining module 220 may be configured to obtain local input functions of at least one overlapping scanning area based on the plurality of local input functions. More descriptions of obtaining local input functions of at least one overlapping scanning area based on the plurality of local input functions may be found in operation 320 of FIG. 3 and related descriptions thereof.

The determination module 230 may be configured to determine a continuous input function of at least a portion of a target scanning area based on the local input functions of the overlapping scanning area. More descriptions of determining a continuous input function may be found in operation 330 of FIG. 3 and descriptions thereof.

It should be understood that a system and its modules shown in FIG 2 and may be realized by using a variety of approaches. For example, the system and its modules may be realized through hardware, software, or a combination of the software and the hardware. The system and its modules of the present disclosure may be implemented not only by hardware circuits such a very largescale integration or a very large scale gate array, a semiconductor such as a logic chip, a transistor, etc., or a programmable hardware device such as a field-programmable gate array, a programmable logic device, etc., but also by software executed by various types of processors, for example, a combination of the above hardware circuits and software (e.g., firmware ).

It should be noted that the above descriptions of the system and its modules are merely provided for the purpose of description, as a schematic, and does not limit the present disclosure to the scope of the cited embodiments. It may be understood that, those skilled in the art, after understanding the principle of the system, may make arbitrary combinations to various models or form sub-systems connected to other module, without departing from this principle.

FIG. 3 is a flow diagram illustrating an exemplary process for imaging according to some embodiments of the present disclosure. In some embodiments, process 300 may be performed by the processing device 120 or the system for imaging 200. For example, process 300 may be stored in a storage device (e.g., the storage device 140, a storage unit of the processing device 120) in forms of programs or instructions, and process 300 may be realized when the processor or the module shown in FIG. 2 executes the program or instructions. In some embodiments, process 300 may be implemented by using one or more additional operations not descritable position below, and/or not by the one or more operations are completed. Further, a sequence of operations as shown in FIG. 3 is not limited.

In 310, a plurality of local input functions of an object may be obtained. Each of the plurality of local input functions may correspond to or be of a scanning area of the object. A local input function may be obtained based on a scan (also refered to as a local scan or a sub-scan) of one of a plurality of table positions.

In some embodiments, the plurality of table positions may include a target table position corresponding to a target scanning area and one or more reference table positions corresponding to one or more reference scanning areas. Two adjacent table positions in the plurality of table positions may have or correspond to an overlapping scanning area. As used herein, the overlapping scanning area refers to a portion of the object is scanned for twice during the scans of the adjacent table positions. In some embodiments, operation 310 may be performed by the processing device 120 or the first obtaining module 210.

In some embodiments, the length of the object may be greater than the axial FOV of a PET scanner, a full scan of the object may be performed based on the plurality of table positions. The full scan of the object may include multiple sub-scans (also referred to as a local scan) each of which corresponds to one of the plurality of table positions. Each of the plurality of table positions may correspond to or define a scanning range (also referred to as a scanning area) of the object. For example, due to a range limitation of a detector in a longitudinal direction during a whole-body or half-body scan, the full scan including the plurality of table positions may be performed.

The scanning area (i.e., the target scanning area) corresponding to the target table position may cover a specific portion (e.g., tissue, organ, etc., or a region of interest) of the object. For example, a scanning area corresponding to a cardiac table position may cover the heart area. As another example, a scanning area corresponding to a bladder table position may cover the bladder area.

A reference table position may include a table position excepting the target table position in the plurality of table positions. For example, the reference table position may include a first reference table position, a second reference table position, etc. The reference table position may be adjacent or not adjacent to the target table position.

The scanning areas corresponding to two adjacent table positions in the plurality of table positions may have the overlapping scanning area. In other words, the two adjacent table positions in the plurality of table positions may have the overlapping scanning area. As used herein, the overlapping scanning area between the two adjacent table positions may include a portion of the scanning area corresponding to one of the two adjacent table positions and a portion of the scanning area corresponding to another one of the two adjacent table positions. The portion of the scanning area corresponding to one of the two adjacent table positions and the portion of the scanning area corresponding to the another one of the two adjacent table positions may be overlapped. The portion of the scanning area corresponding to one of the two adjacent table positions overlapping the portion of the scanning area corresponding to the another one of the two adjacent table positions refers to that the portion of the scanning area corresponings to one of the two adjacent table positions and the overlapped portion of the scanning area corresponding to the another one of the two adjacent table positions covers the same portion of the object when the object is scanned on the two adjacent table positions. In other words, the same portion of the object may be scanned twice in the sub-scans of the two adjacent table positions. A scanning area corresponding to the latter one of the two adjacent table positions (except the first and last table position) may include the percentage R of a scanning area corresponding to a previous table position of the two adjacent table positions and the percentage R of a scanning area corresponding to a subsequent table position of the latter one of the two adjacent table positions. The percentage R may also be referred to as an overlapping scanning area percentage R, and may be set based on experience and/or demand. For example, the percentage R may be 25%, 30%, 50%, etc. In some embodiments, the percentage of overlapping scanning area in each table position among multiple table positions may be different. For example, the percentage of overlapping scanning area in the corresponding scanning area of the target table position is 50%, the percentage of overlapping scanning area in the corresponding scanning area of the first reference table position is 35%, and the percentage of overlapping scanning area in the corresponding scanning area of the second reference table position is 40%.

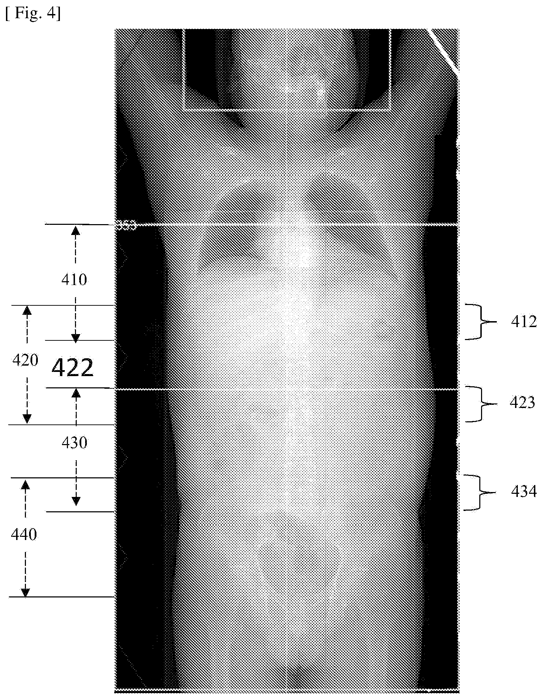

For example, as shown in FIG. 4, a scanning area 410 corresponding to the target table position (e.g., a cardiac table position) has an overlapping scanning area 412 (i.e., a first portion of the scanning area 410) with a scanning area 420 corresponding to the first reference table position, and the scanning area 420 corresponding to the first reference table position has an overlapping scanning area 412 (i.e., a first portion of the scanning area 420) with the scanning area 410 corresponding to the target table position. The scanning area 420 corresponding to the first reference table position has an overlapping scanning area 423 (i.e., a second portion of the scanning area 420) with the scanning area 430 corresponding to the second reference table position, and the scanning area 430 corresponding to the second reference table position has an overlapping scanning area 423 (i.e., a first portion of the scanning area 430) with the scanning area 420 corresponding to the first reference table position. The scanning area 430 corresponding to the second reference table position has an overlapping scanning area 434 (i.e., a second portion of the scanning area 430) with the scanning area 440 corresponding to a third reference table position, and the scanning area 440 corresponding to the third reference table position has an overlapping scanning area 434 (i.e., a first portion of the scanning area 440) with the scanning area 430 corresponding to the second reference table position, etc. The scanning area 420 includes a portion 422 that is not overlapped with the scanning area 410 and the scanning area 430.

In some embodiments, the length of the object may be greater than an axial FOV (Field of View) length of the PET scanner. The axial FOV length of the PET scanner may be thea length of the image range covered by the PET scanner in an axial direction. The FOV length may determine a size of the field of view of the image and a range of anatomical structures captured. In some embodiments, when the length of the object is greater than the axial FOV (Field of View) length of the PET scanner, the object may be scanned for the plurality of table positions to obtain the scanning data (e.g., coincidence event data) corresponding to each of the plurality of table positions. The scanning data corresponding to each of the plurality of table positions may be used to determine one or more images, a local input function, etc., corresponding to the one of the plurality of table positions or the scanning area correpsonding to the one of the plurality of table positions.

In some embodiments, the length of the object may be equal to the axial FOV length of the PET scanner. In some embodiments, the length of the object may be less than the axial FOV length of the PET scanner.

An input function is a time activity curve and/or a standardized uptake value (SUV) curve of drug in a portion of voxel within a scanning range. For example, an input function may include the time activity curve and/or the standardized uptake value (SUV) curve of radionuclide in the voxel in an area of descending aorta within the scanning range. The input function may be used for the reconstruction of a subsequent parameter image, calculation of blood flow to the organ, and other computational processes. In some embodiments, a horizontal coordinate of an input function curve is time and a vertical coordinate of the input function curve is activity of the radionuclide. In some embodiments, the horizontal coordinate of the input function curve is time and the vertical coordinate of the input function curve is a standardized uptake value (SUV) of the drug. It should be noted that the input function is input information to a parameter imaging model and may be obtained by analyzing blood information in PET images obtained by the process for imaging. For example, blood pools such as the aorta, left ventricle, etc., are used as an input source of the image information, such that the input function is obtained.

For one of the plurality of table positions, a local input function of a scanning area corresponding to the table position may be obtained based on scanning data obtained in the sub-scan of the one of the plurality of table positions. The local input function of the scanning area may be a portion of a time activity curve and/or a standardized uptake value (SUV) curve of a drug in a portion of voxel within the scanning area in a time period when the sub-scan of the table position is performed. As used herein, a local input function refers to a portion of a continuous input function in a timer period that is a portion of a whole scan time period.

The plurality of table positions in which the object is located may correspond to the plurality of local input functions. In some embodiments, each table position may correspond to a local input function or a portion thereof. In other words, a local input function may correspond to a time period during which a scan of a table position is performed.

In some embodiments, the first obtaining module 210 may obtain each of the plurality of local input functions in a corresponding time period by scanning the object at a table when the table is at one of the plurality of table positions. For example, as shown in FIG. 4, the target local input function of a target scanning area corresponding to the target table position (e.g., cardiac table position) in a time period t₀ - t₁ may be obtained by scanning the scanning area 410 in the time period t₀ - t₁. The first reference local input function (also referred to as first local input function) of the first scanning area corresponding to the first reference table position in a time period t₁ - t₂ may be obtained by scanning the scanning area 420 in the time period t₁ - t₂. The second reference local input function of the second scanning area corresponding to the second reference table position in a time period t₂ - t₃ may be obtained by scanning the scanning area 430 in the time period t₂ - t₃.

In some embodiments, the first obtaining module 210 may obtain the local input functions of the scanning areas corresponding to the plurality of table positions through the following operations. For one table position of the plurality of table positions, the first obtaining module 210 may obtain the scanning data acquired based on a scan (also referred to as a local scan or a sub-scan) of the table position where the table placing the object is located. The first obtaining module 210 may determine the local input function corresponding to the table position based on the scanning data.

Taking PET imaging as an example, in some embodiments, the first obtaining module 210 may obtain the scanning data, for example, raw data stored in a Listmode (list) mode, raw data stored in a Sinogram (chordogram) mode, etc., by scanning the object located in the table position. In some embodiments, the first obtaining module 210 may convert the scanning data into the image data by an approach such as the Radon transform. The image data may include an image sequence including multiple frames. In some embodiments, the first obtaining module 210 may obtain an average activity value of activity values of specific voxels (e.g., voxels within the scanning area of a descending aorta area) from each frame in the image data. The first obtaining module 210 may determine the average activity value as a value of the local input function at a time point of the frame, i.e., the vertical ordinate of an input function curve at the time point of the frame, such that a local input function during the scanning time of the table position may be obtained.

In some embodiments, the first obtaining module 210 may determine the local input function of the scanning area corresponding to the table position through other approaches based on the scanning data. For example, the scanning data may be input into a trained machine learning model to obtain the corresponding local input function.

In some embodiments, the first obtaining module 210 may obtain the local input function of the object corresponding to the scanning area defined by one of the plurality of table positions of the object through other approaches. For example, the local input function may be obtained by a process of arterial continuous blood collection. As another example, the local input function may be obtained based on the population information-based input function.

In some embodiments, a plurality of rounds of scanning may be performed on the object. Each round of scanning in the plurality of rounds of scanning may include the sub-scans of the plurality of table positions. In some embodiments, in each round of the plurality of rounds of scanning, the first obtaining module 210 may obtain the local input function corresponding to the scanning area defined by one of the plurality of table positions of the object to generate a continuous input function within the round of scanning.

In 320, local input functions corresponding to overlapping scanning areas may be obtained. In some embodiments, operation 320 may be performed by the processing device 120 or the second obtaining module 220.

In some embodiments, the second obtaining module 220 may obtain the local input functions (also referred to as overlapping input functions) corresponding to the at least one overlapping scanning area based on the plurality of local input functions. For an overlapping scanning area between two adjacent table positions, the second obtaining module 220 may determine a local input function of the overlapping scanning area corresponding to one of the two adjacent table positions by intercepting the local input function corresponding to the scanning area defined by the one of the two adjacent table position and determine a local input function of the overlapping scanning area corresponding to another one of the two adjacent table position by intercepting the local input function corresponding to the scanning area defined by the another one of the two adjacent table position.. The overlapping input functions may be served as correction information for difference of time and intensity during a multi-table position scanning.

As used herein, an overlapping scanning area between two adjacent table positions may correspond to two local input functions obtained based on scans of the two adjacent table positions.

It should be noted that for each of the at least one overlapping area between two adjacent table positions, the overlapping input functions may include a segment of a first local input function (also referred to as a local input function segment) of a portion of the scanning area corresponding to one of the two adjacent table positions and a segment of a second local input function (also referred to as a local input function segment) of a portion of the scanning area corresponding to another one of the two adjacent table positions. The first local input function may correspond to the scanning area corresponding to one of the two adjacent table positions, and correspond to the time period for performing the sub-scan of one of the two adjacent table positions. The second local function may correspond to the scanning area corresponding to the another one of the two adjacent table positions, and correspond to the time period for performing the sub-scan of the another one of the two adjacent table positions. The overlapping input functions of the overlapping scanning area may be obtained based on the first local input function of the scanning area corresponding to one of the two adjacent table positions and the second local input function of the scanning area corresponding to the another one of the two adjacent table positions.

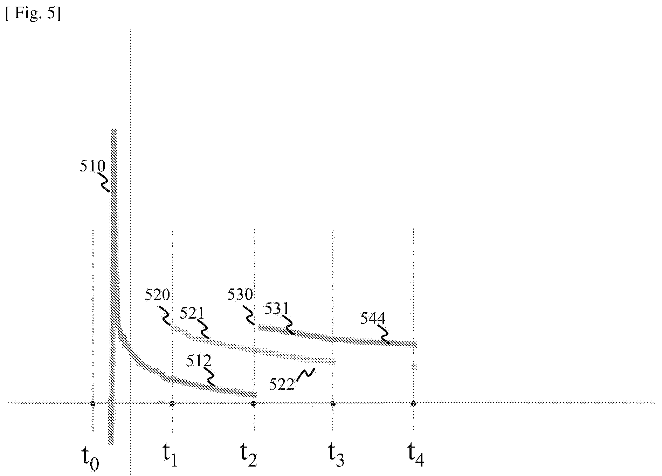

For example, as shown in FIG. 5, local input functions 510, 520, and 530 may correspond to three overlapping scanning areas including a first overlapping area, a second overlapping area, and a third overlapping area (e.g., area 412, area 423, and area 434 shown in FIG. 4), respectively. The first overlapping area is between the target table position and a first reference table position, the second overlapping area is between the first reference table position and the second reference table position, and the third overlapping area is between the second reference table position and the third reference table position. The data collection time for the target table position is time period t₀ - t₁, and a portion of the local input function 510 corresponding to the time period t₀ - t₁ may be obtained. The data collection time for the first reference table position is time period t₁ - t₂, and a portion (i.e., portion 512) of the local input function 510 corresponding to the time period t₁ - t₂ may be obtained , and portion 521 of the local input function 520 corresponding to the same time period may be obtained. The data collection time for the second reference table position is the time period t₂ - t₃, under which portion 522 of the local input function 520 corresponding to the time period t₂ - t₃ may be obtained, and portion 531 of the local input function 530 of the third overlapping area corresponding to the same time period may be obtained.

The local input function 510 of the first overlapping area between the target table position and the first reference table position includes two portions or parts (i.e., a first portion and a second portion) in two time periods t₀ - t₁ and t₁ - t_{2.} In some embodiments, the first portion of the local input function 510 in the time periods t₀ - t₁ may be obtained based on the scan of the target table position. For example, the first portion of the local input function 510 may be determined based on the scan data of the first overlapping area acquired in the scan of the target table position in the time period t₀ - t_{1.} In some embodiments, the first portion of the local input function 510 may be determined by intercepting the target local input function 510 The second portion of the local input function 510 in the time period t₁ - t₂ may be obtained based on the scan of the first reference table position. For example, the second portion of the local input function 510 may be determined based on the scan data of the first overlapping area acquired in the scan of the first reference table position in the time period t₁ - t_{2.} As another example, the second portion of the local input function 510 may be determined by intercepting the target local input function 510

The local input function 520 of the second overlapping area between the first reference table position and the second reference table position may include two portions or parts (e.g., a first portion and a second portion) in two time periods t₁ - t₂ and t₂ - t_{3.} In some embodiments, the first portion of the local input function 520 in the time periods t₁ - t₂ may be obtained based on the scan of the first reference table position. In some embodiments, the first portion of the local input function 520 may be determined by intercepting the local input function 520 In some embodiments, the second portion of the local input function 520 in the time periods t₂ - t₃ may be obtained based on the scan of the second reference table position. In some embodiments, the second portion of the local input function 520 may be determined by intercepting the local input function 520

The local input function 530 of the second overlapping area between the second reference table position and the third reference table position may include two portions or parts (e.g., a first portion and a second portion) in two time periods t₂ - t₃ and t₃ - t_{4.} In some embodiments, the first portion of the local input function 530 in the time periods t₂ - t₃ may be obtained based on the scan of the second reference table position. In some embodiments, the first portion of the local input function 530 may be determined by intercepting the local input function 530 The second portion of the local input function 530 in the time periods t₃ - t₄ may be obtained based on the scan of the third reference table position. In some embodiments, the second portion of the local input function 530 may be determined by intercepting the local input function 530

It is noted that, for simplicity of presentation, FIG. 5 of the present disclosure is drawn based on the overlapping scanning area with a ratio R of 50%. In other words, first 50% of the scanning area corresponding to each table position (excepting a first table position and last table position) overlaps with the scanning area corresponding to a previous table position, and second 50% of the scanning area overlaps with the scanning area corresponding to a subsequent table position.

In 330, a continuous input function of at least a portion of the target scanning area correpsonding to the target table postion may be determined based on the information of local input functions corresponding to the overlapping scanning area and reference local input functions corresponding to one or more reference scanning areas defined by the one or more reference table positions. In some embodiments, operation 330 may be performed by the processing device 120 or the determination module 230. As used herein, the continuous input function of at least a portion of the target scanning area refers to an input function of the at least a portion of the target area during a full time for performing the scans of the plurality of table positions. The target table position may be such that when the table with the object is located at the target table position, the target scanning area is located at a center region of a scanning region of the scanning device, such that the scanning region covers the target scanning area.

The continuous input function of at least a portion of the target scanning area may be a complete and uninterrupted input function corresponding to the target scanning area during the full scanning time for the plurality of table positions. The full scanning time may include multiple time periods each of which corresponds to one of the plurality of table positions. For example, as shown in FIG. 5, a time period t₀ - t₄ is a full scanning time that corresponds to the complete uninterrupted input function of the target table position. The local input function of at least a portion of the target scanning area (e.g., the first overlapping scanning area 412 as shown in FIG. 4) during the initial time period (e.g., time period t₀ - t₁ as shown in FIG. 5) may be obtained based on the scan of the target table position in the first time period (e.g., the time period t₀ - t₁ as shown in FIG. 5). In some embodiments, the determination module 230 may obtain additional local input functions of at least a portion of the target scanning area (e.g., the first overlapping scanning area 412 as shown in FIG. 4) during other time periods (e.g., the first time period, the second time period, etc.) based on the overlapping input functions (i.e., local input functions of overlapping scanning areas) , such that the continuous input function of at least a portion of the target scanning area (e.g., the first overlapping scanning area 412 as shown in FIG. 4) during the full scanning time for the plurality of table positions may be obtained.

For example, as shown in FIG. 5, the continuous input function of at least a portion of the target scanning area (e.g., the first overlapping scanning area 412 as shown in FIG. 4) in the time period t₀ - t₄ includes Part A: the target local input function in the time period t₀ - t₁, Part B: a target local input function in the time period t₁ - t_{2,} and Part C: a target local input function in the time period t₂ - t₄. The determination module 230 may obtain Part A based on the scan of the target table position in the time period t₀ - t₁ directly. The determination module 230 may obtain Part B based on the scan of the first table position (also referred to as first reference table position) in the time period t₁ - t₂ directly, i.e. the input function segment 512. The determination module 230 may obtain Part C based on the overlapping input functions (i.e., local input functions) of the overlapping scanning areas between adjacent table positions among the plurality of table positions, for example, the first overlapping scanning area, the second overlapping scanning area, etc. In some embodiments, part A may be a local input function of the first overlapping scanning area between the target table position and the first reference table position in the initial time period for the scan of the target table position; part B may be a local input function of the first overlapping area between the target table position and the first reference table position in the first time period for the scan of the first reference table position. In some embodiments, part A may be a local input function of a non-overlapping scanning area between the target table position and the first reference table position in the initial time period for the scan of the target table position; part B may be a local input function of the non-overlapping area between the target table position and the first reference table position in the first time period for the scan of the first reference table position.

In some embodiments, the at least one overlapping scanning area may include a first overlapping scanning area between a table position and a previous table position and a second overlapping scanning area between the table position and a latter table position, and the determining the continuous input function corresponding to the target scanning area may include determining a ratio of a local input function of the first overlapping scanning area and a local input function of the second overlapping scanning area in a first time period for a scan of the table position, determining at least one part of the continuous input function based on the ratio and a local input function of the second overlapping scanning area in a second time period for a scan of the latter table position; and obtaining the continuous input function based on the at least one part of the continuous input function.

For example, the determination module 230 may determine a ratio of the local input function (also referred to as a first local input function) of the first overlapping scanning area between the target table position and the first reference table position scanned during the first time period (e.g., time period t₁ - t₂) and the local input function (also referred to as a second local input function) of the second overlapping scanning area between the first table position and the second reference table position scanned during the first time period (e.g., time period t₁ - t₂). The determination module 230 may determine the target local input function of at least a portion of the target scanning area (e.g., the first overlapping scanning area 412 as shown in FIG. 4) during the second time period (e.g., the time period t₂ - t₃) based on the ratio of the first local input function and the second local input function. For example, the determination module 230 may determine the target local input function of at least a portion of the target scanning area (e.g., the first overlapping scanning area 412 as shown in FIG. 4) during the second time period (e.g., time period t₂ - t₃) based on the ratio of the first local input function and the second local input function and a third local input function of the second overlapping scanning area in the second time period (e.g., the time period t₂ - t₃).

The local input function of the first overlapping scanning area corresponding to the third time period (e.g., the time period t₃ - t₄) may be determined based on the third local input function of the first overlapping scanning area between the first reference table position and the second reference table position scanned during the second time period (e.g., time period t₂ - t₃), and the fourth local input function of the second overlapping scanning area between the first reference table position and the second reference table position in the second time period (e.g., the time period t₂ - t₃) of the second reference table position scanned during time period t₃ -t_{4.} For example, the determination module 230 may determine a ratio of the third local input function and the fourth local input function. The determination module 230 may determine the local input function of the first overlapping scanning area corresponding to the third time period (e.g., t₃ - t₄) based on the ratio of the third local input function and the fourth local input function. As a futher example, the determination module 230 may determine the local input function of the first overlapping scanning area corresponding to the third time period (e.g., t₃ - t₄) based on a fifth local input function of the second overlapping scanning areas in the third time period (e.g., the time period t₃ - t₄) and the ratio of the third local input function and the fourth local input function.. Then the local input function of the first overlapping scanning area in the second time period and in the third time period may be obtained.

In some embodiments, the at least one reference table position may include a first reference table position adjacent to and subsequent to the target table position and a second reference table position adjacent to and subsequent to the first reference table position. The at least one overlapping scanning area may include a first overlapping scanning area and a second overlapping scanning area. The first overlapping scanning area may be between the target table position and the first reference table position. The second overlapping scanning area may be between the first reference table position and the second reference table position In some embodiments, the determination module 230 may obtain at least a portion of the continuous input function based on the local input functions (i.e., overlapping input functions) of the first overlapping scanning area and the second overlapping scanning areathrough the following operations.

The determination module 230 may obtain the first local input function in the first time period. The first time period may be a time period for scanning the first overlapping scanning area, and the target local input function may be a local input function corresponding to the target table position. For example, the first local input function is an input function segment 512 of the target local input function 510 during the time period t₁ - t₂.

The determination module 230 may obtain a second local input function of the second overlapping scanning area during the first time period. For example, the second local input function is a local input function 521 obtained during the time period t₁ - t₂.

Further, the determination module 230 may obtain a first ratio of the first local input function to the second local input function.

In some embodiments, the determination module 230 may obtain an average value of the ratios. Each of the ratios may be of the first local input function to the second local input function at mulitple time points during the first time period, and determine the average value of the ratios as the first ratio. In other words, the first local input function may include multiple values at the multiple time points during the first time period, and the second local input function may include multiple values at the multiple time points during the first time period. The determination module 230 may determine a ratio of one of the multiple values of thefirst local input function at a time point and one of the multiple values of the second local input function at the same time point. Then the determination module 230 may determine the average value of the multiple ratios. In some embodiments, the determination module 230 may designate a maximum value, a minimum value, or a median value, etc., among the multiple ratios as the first ratio.

The determination module 230 may obtain the at least a portion of the continuous input function during a second time period based on the first ratio and third local input function of the second overlapping scanning area during the second time period. For example, as shown in FIG. 5, the at least a portion of the continuous input function at the time period t₂ - t₃ is obtained based on the first ratio and the third local input function (i.e., the local input function 522) of the first reference local input function 520 (or the second overlapping scanning area) during the time period t₂ - t₃.

In some embodiments, the determination module 230 may obtain the at least a portion of the continuous input function during the second time period based on the following equation (1): ${P}_{1} \left({t}_{23}\right) = mean \left({f}_{12}\left({t}_{12}\right)\right) \times {P}_{2} \left({t}_{23}\right) ,$ Where f₁₂(t₁₂)= P₁(t₁₂)/ P₂(t₁₂), f₁₂(t₁₂) denotes the ratios of the first local input function to the second local input function during the first time period (e.g.,the time period t₁ - t₂); mean (f₁₂ (t₁₂)) denotes an average value of the ratios of the first local input function to the second local input function during the first time period (e.g.,the time period t₁ - t₂). P₂ (t₂₃) is the third local input function of the second overlapping scanning area corresponding to the first reference table position, i.e., the input function segment during the second time period (e.g., the time period t₂ - t₃). P₁ (t₂₃) is at least one portion of the continuous input function corresponding to the target table position obtained during the second time period (e.g., the time period t₂ - t₃).

In some embodiments, the at least one reference table position may further include the third reference table position. The overlapping scanning area may further include a third overlapping scanning area between the second reference scanning area and a third reference scanning area defined by the third reference table position. The third overlapping scanning area may correspond to the third time period during which the third overlapping scanning area is scanned. The determination module 230 may obtain the at least a portion of the continuous input function based on the local input functions of the first overlapping scanning area, the second overlapping scanning area, and the third overlappiong scanning areaby the following operations.

The determination module 230 may obtain the third local input functionof the second overlapping scanning area in the second time period. The second time period may be a time period for scanning the second reference scanning area corresponding to the second reference table position.

The determination module 230 may obtain a fourth local input function of the third overlapping scanning area during the second time period. The second reference local input function may be an input function corresponding to the second reference table position. For example, the fourth local input function is a local input function 531 of the second reference local input function 530 obtained during the time period t₂ - t₃.

Further, the determination module 230 may obtain a second ratio of the third local input function to the fourth local input function.

In some embodiments, the determination module 230 may obtain the average value of the ratios. Each ratio may be of the third local input function to the fourth local input function at each time point during the second time period, and determine the average value of the ratios as the second ratio. In other words, the third local input function may include multiple values at time points during the second time period, and the fourth local input function may include multiple values at the time points during the second time period. The determination module 230 may determine a ratio of one of the multiple values of the third local input function at a time point and one of the multiple values of the fourth local input function at the time point. Then the determination module 230 may determine the average value of the multiple ratios. In some embodiments, the determination module 230 may designate a maximum value, a minimum value, or a median value, etc., among the multiple ratios as the second ratio.

Finally, the determination module 230 may obtain the at least a portion of the continuous input function during the third time period based on the second ratio and a fifth local input function of the third overlapping scanning area during the third time period. For example, the fifth local input function of the third overlapping scanning area is thre local input function 544 during the time period t₃ -t₄.

In some embodiments, the determination module 230 may obtain the at least a portion of the continuous input function at the third time period based on the first ratio, the second ratio, and the fifth local input function of the third overlapping scanning area at the third time period. For example, the determination module 230 may obtain the at least a portion of the continuous input function at the third time period based on the following equation (2). ${P}_{1} \left({t}_{34}\right) = mean \left({f}_{12}\left({t}_{12}\right)\right) \times mean \left({f}_{23}\left({t}_{23}\right)\right) \times {P}_{3} \left({t}_{34}\right) ,$ where f₁₂(t₁₂)= P₁(t₁₂)/ P₂(t₁₂), f₁₂(t₁₂) denotes the ratios of the first local input function to the second local input function during the first time period (e.g.,the time period t₁ - t₂); mean(f₁₂ (t₁₂ )) represents the average value of the ratios of the first local input function to the second local input function in the first time period, e.g.,the time period t₁ -t₂; f₂₃(t₂₃)= P₂(t₂₃)/ P₃(t₂₃), f₂₃(t₂₃) denotes the ratios of the third local input function to the fourth local input function in the second time period; mean(f₂₃ (t₂₃ )) represents the average value of the ratios of thethird local input function to the fourth local input functionn in the second time period, e.g., the time period t₂ - t₃; p₃ (t₃₄ ) is the fifth local input function of the third overlapping scanning area , i.e. the input function segment for the third time period, e.g., the time period t₃ - t₄. P₁ (t₃₄) is at least a part of the continuous input function corresponding to the target table position in the time period t₃ - t₄.

In some embodiments, the determination module 230 may determine a ratio of the local input functions corresponding to each of the overlapping areas of each two adjacent table positions. In some embodiments, the determination module 230 may determine the at least a portion of the continuous input function based on the ratio and the local input function of the last overlapping scanning area. The last overlapping scanning area is between the last table position and/or the table position that is farthest from the target table position and a previous table position of the last table position. In some embodiments, the at least a portion of the continuous input function (e.g., a last portion of the continuous input function) may be obtained according to the following equation (3): ${P}_{1} \left({t}_{n , n + 1}\right) = mean \left({f}_{12}\left({t}_{12}\right)\right) \times mean \left({f}_{23}\left({t}_{23}\right)\right) \times … \times mean \left({f}_{n - 1 , n}\left({t}_{n - 1 , n}\right)\right) \times {P}_{n} \left({t}_{n , n + 1}\right) ,$ where f₁₂(t₁₂)= P₁(t₁₂)/ P₂(t₁₂), f₁₂(t₁₂) denotes the ratios of the first local input function to the second local input function during the first time period (e.g.,the time period t₁ - t₂); mean(f₁₂ (t₁₂ )) denotes an average value of the ratios of the first local input function to the second local input function during the first time period, e.g., the time period t₁ - t₂; f₂₃(t₂₃)= P₂(t₂₃)/ P₃(t₂₃), f₂₃(t₂₃) denotes the ratios of the third local input function to the fourth local input function in the second time period; mean(f₂₃ (t₂₃ )) denotes an average value of the ratios of the third local input function to the fourth local input functionduring the second time period, e.g., the time period t₂ - t₃. Pₙ (tₙ, ₙ₊₁) is a local input function of the n^{th} overlapping scanning area,. P₁ (t_{n,n+1} ) is at least a portion of the continuous input function corresponding to the target table position obtained during the time period tₙ - tₙ₊₁. The processing device 120 may determine multiple portions of the continuous input function during different time periods according to equation (3).

In other words, for each two adjacent table positions including a current table position and a later table position, the processing device 120 may determine a ratio of the local input functions (or input function segments) corresponding to the overlapping scanning area of the current table position and the previous table position. The processing device 120 may determine the input function segment of a non-overlapping area of the current table position based on the ratio and the input function segment of the non-overlapping area of the later table position. The processing device 120 may update the current table position using the previous table position of the current table position, and the updated current table position may be the later table position, and the previous table position may be the current table position. Then the processing device 120 may repeat to determine the input function segment of a non-overlapping area of the current table position until the current table position is the adjacent table position of the target table position. The processing device 120 may determine the input function segment of the non-overlapping area of the target table position with the adjacent table position based on the ratio of the local input functions (or input function segments) corresponding to the overlapping scanning area of the target table position and the later table position of the target table position and the input function segment of the non-overlapping area of the later table position of the target table position.

In some embodiments, the determination module 230 may stitch the target local input function and the determined portions of the continuous input function (i.e., one or more input function segments corresponding to different time periods), such that the continuous input function may be analogous to a final drug concentration curve obtained in a plurality of blood collections. In some embodiments, the determination module 230 may perform curve smoothing on a spliced continuous input function to ensure a coherence of the curve.

In some embodiments, the processing device 120 may perform a plurality of rounds of scanning on the object. Each round in the plurality of rounds of scanning may include the scans of a plurality of table positions. For example, each round of the plurality of rounds of scanning may include scans of the target table position, the first reference table position, the second reference table position, ......, and the last reference table position, in that order, from the head to the foot direction. Alternatively, each round of the plurality of rounds of scanning may include scanning the last reference table position, ......, the second reference table position, the first reference table position, and the target table position, in that order, from a foot-to-head direction.

In some embodiments, the processing device 120 may determine the continuous input function corresponding to the target table position in the round of scanning based on the information of overlapping input functions and the reference local input function corresponding to the at least one reference table position within the same scanning round. For example, both a first round of scanning and a second round of scanning may include the scans of the target table position, the first reference table position, the second reference table position, ......, and the last reference table position in a head-to-toe direction. The processing device 120 may determine the continuous input function of the first round of scanning based on the information of overlapping input functions obtained from the first round of scanning, and the processing device 120 may determine the continuous input function of the second scan round based on the information of overlapping input functions obtained from the second round of scanning.

It should be noted that the above descriptions of process 300 are merely provided for the purpose of example and description, which are not intended to limit the scope of application of the present disclosure. For those skilled in the art, various amendments and variations may be made to the process 300 under the teaching of the present disclosure. However, these amendments and variations remain within the scope of the present disclosure.

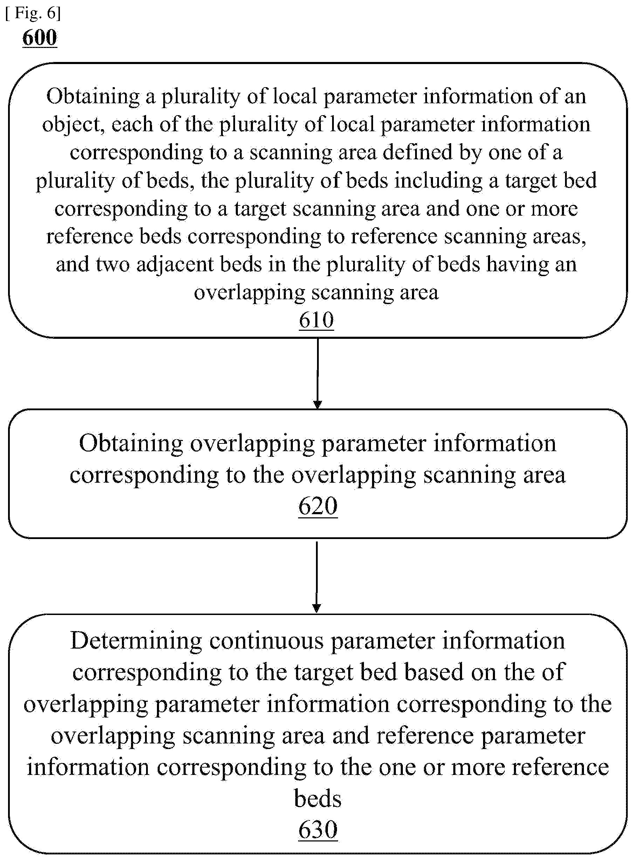

FIG. 6 is a flowchart illustrating an exemplary process for imaging according to some embodiments of the present disclosure. In some embodiments, process 600 may be performed by the processing device 120 or the system for imaging 200. For example, process 600 may be stored in a storage device (e.g., the storage device 140, the storage unit of processing device 120) in forms of programs or instructions, and process 600 may be realized when the processor or the module shown in FIG. 2 executes the programs or instructions. In some embodiments, the process 600 may be implemented by using one or more additional operations not descritable position below, and/or not by the one or more operations below. Further, the order of operations as shown in FIG. 6 is not limited.

In 610, a plurality of local parametric information of an object may be obtained. Each of the plurality of local parametric information may correspond to a scanning area defined by one of a plurality of table positions. The plurality of table positions may include a target table position corresponding to a target scanning area and one or more reference table positions corresponding to reference scanning areas, and two adjacent table positions in the plurality of table positions may have an overlapping scanning area. In some embodiments, operation 610 may be performed by the processing device 120 or the first obtaining module 210.

More descriptions regarding the plurality of table positions, the scanning areas corresponding to adjacent table positions in the plurality of table positions, and the overlapping scanning areas, may be found in operation 310 and related descriptions thereof.

Parametric information refers to various parameters and related data used in PET to describe the behavior and features of radiotracer in the organism. The parametric information includes an input function, a target lesion curve, image data, a kinetic parameter, etc.

The target lesion curve may reflect the presence and a feature of the lesion. In PET imaging, the radiotracer is injected into the body and then collects in the tissue, and the focal areas usually have different biological activity or metabolic states, resulting in different concentration changes of the tracer in the focal areas compared to the surrounding normal tissue. Some quantitative and qualitative information, such as a level of metabolic activity, blood flow, and a degree of receptor binding, may be obtained from the lesion curve by observing features, such as curve morphology, peak value, and time delay of the lesion area.

The image data may include an image sequence including a plurality of images at a plurality of time points in the PET imaging. The image data may be configured to observe and analyze dynamic changes in metabolism and blood circulation in the living organisms.

The Kinetic parameter may be used to describe a rate of delivery and the metabolism of a radiolabel within the tissue or the lesion, for example, k1 (transport rate constant), k2 (reaction rate constant), Vd (volume of distribution), BP (binding potential), SUV (standard uptake value), etc. The kinetic parameters may provide more comprehensive and quantitative information that may help reveal the biological features, metabolic activity, and receptor binding of tissue or lesion.

The parametric information is important for quantitative assessment of metabolic activity of the lesion, a degree of uptake, and for disease diagnosis and treatment monitoring. Parametric imaging(generating images with the parametric information) is more sensitive to the change of drug distribution in vivo. It can be used to locate small lesions and discriminate the edge of the lesion. This characteristic can be used to tract the lesion growth or effect of the drug in a long term.

In some embodiments, the plurality of table positions where the object is located corresponds to the plurality of local input functions. In some embodiments, each table position of the plurality of table positions may correspond to the local input function or a portion thereof.

In some embodiments, the first obtaining module 210 may obtain the plurality of local parametric information defined by the plurality of table positions of the object through a process similar to operation 310.

For example, for each table position in the plurality of table positions, the first obtaining module 210 may obtain the scanning data of the object located in that table position; and determine the parametric information corresponding to the table position based on the scanning data.

In 620, overlapping parametric information corresponding to the overlapping scanning areas may be obtained. In some embodiments, operation 620 may be performed by the processing device 120 or the second obtaining module 220.

In some embodiments, the first obtaining module 220 may obtain the overlapping parametric information corresponding to the overlapping scanning area by a process similar to operation 320.

In 630, continuous parametric information corresponding to the target table position may be determined based on the overlapping parametric information corresponding to the overlapping scanning areas. In some embodiments, operation 630 may be performed by processing device 120 or the determination module 230.

The continuous parametric information corresponding to the target table position may be the complete uninterrupted parametric information corresponding to the target table position during the full scanning time for the plurality of table positions. The full scanning time may include multiple time periods each of which corresponds to one of the plurality of table positions. For example, a time period t₀ - t₄ corresponds to the complete uninterrupted parametric information of the target table position. Through operation 610, the first obtaining module 210 may only obtain interrupted parametric information of a plurality of scanned target table positions during time periods, e.g., target local parametric information corresponding to the target table positions during the time period t₀ - t₂ and target local parametric information corresponding to the target table position during the time period t₁ - t₃. In some embodiments, the determination module 230 may obtain the parametric information corresponding to the target table position during other scanning time periods based on the overlapping parametric information such that the continuous parametric information corresponding to the target table position in the full scanning time periods for the plurality of table positions may be obtained.

In some embodiments, the determination module 230 may determine the continuous parametric information corresponding to the target table positions through an approach similar to operation 330.

For example, in some embodiments, the determination module 230 may determine a ratio of the local parametric information of the adjacent table positions corresponding to each overlapping area; and determine at least one part of the continuous parametric information based on the ratio, and the local parametric information corresponding to the reference area (excepting the overlapping scanning area) of the scanning area corresponding to the last reference table position in the one or more reference table positions.

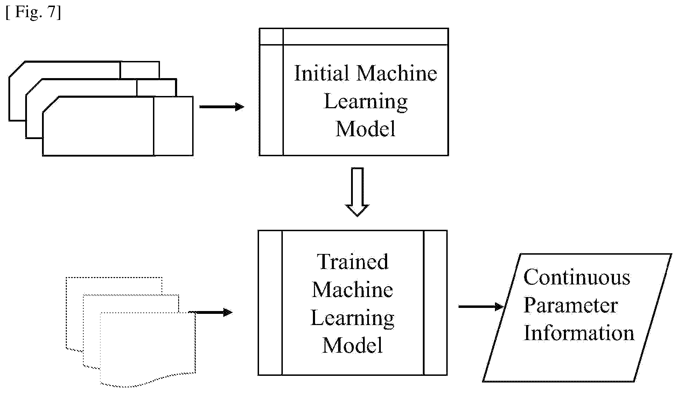

FIG. 7 is an exemplary schematic diagram illustrating an exemplary machine learning model according to some embodiments of the present disclosure.

In some embodiments, the processing device 120 may use the trained machine learning model to determine the continuous parameter information corresponding to the target table position.

For example, as shown in FIG. 7, an input of the trained machine learning model may include a plurality of local parameter information limited by the plurality of table positions of the object, e.g., a plurality of local input function curves, target lesion curves, continuous imaging, kinetic parameters, etc. An output of the trained machine learning model may include the continuous parameter information corresponding to the target table position, e.g., a continuous input function corresponding to the target table position, a continuous target lesion curve corresponding to the target table position, continuous imaging corresponding to the target table positions, continuous kinetic parameters corresponding to the target table positions, etc.

In some embodiments, the trained machine learning model may be constructed based on a sequence model. An exemplary sequence model may include, for example, the sequence model such as a recurrent neural network (RNN), a long short-term memory network (LSTM), or any combination thereof. As another example, the machine learning model may include a convolutional neural network, a recurrent neural network, and a fully connected layer. The convolutional neural network processes the local parameter information of each table position of the plurality of table positions separately, and extracts features of each of the plurality of local parameter information, where a size of the convolutional kernel may be set according to the experience or demand, for example, a size of the convolutional kernel may be 3*3. The sequence model processes the features of the local parameter information, extracts sequence features, and the sequence features include a feature relationship between a previous table position and a subsequent table position. The fully connected layer processes the sequence features to determine the continuous parameter information corresponding to the target table position.

In some embodiments, the trained machine learning model may be constructed based on, for example, a support vector machine model, a logistic regression model, a plain Bayesian classification model, a Gaussian distributed Bayesian classification model, a decision tree model, a random forest model, a KNN classification model, a neural network model, and the like.

In some embodiments, the processing device 120 may train an initial machine learning model based on a large number of training samples to update parameters of the machine learning model, such that the trained machine learning model may be obtained. In some embodiments, each training sample of at least a portion of the large number of training samples includes a plurality of sample local parameter information corresponding to the plurality of table positions, e.g., a plurality of sample local input function curves, a plurality of sample local target lesion curves, a plurality of sample local continuous imaging, a plurality of sample local kinetic parameters, etc. Sample labels may be standard continuous parameter information, e.g., standard continuous input function curves, standard continuous target lesion curves, standard continuous imaging, standard continuous kinetic parameters, etc. In some embodiments, the sample labels may be obtained by processing a plurality of sample local parameter information of the plurality of table positions through the process of FIG. 3 and/or FIG. 6.

In some embodiments, the processing device 120 may randomly select a training set from a large number of training samples and perform data augmentation on the training set.

In some embodiments, the processing device 120 may adjust the parameters of the machine learning model to reduce the difference between the predicted continuous parameter information and labels of continuous parameter information.

In some embodiments, the processing device 120 may reflect the difference between the predicted continuous parameter information and the labels of the continuous parameter information by constructing a loss function. The loss function may include a cross-entropy loss function, an average squared loss function, an exponential loss function, a logarithmic loss function, a squared loss function, etc.

In some embodiments, the processing device 120 may perform several times of iterative training on the initial machine learning model of the training set to obtain the trained machine learning model. Iterative training may include calculating a gradient of the loss function, and iteratively updating the parameters of the machine learning model by a gradient descent approach to reduce the difference between the predicted continuous parameter information and labels of continuous parameter information. The gradient descent process may include a standard gradient descent and a stochastic gradient descent, etc. Various learning rate decay strategies may be adopted in iterative training, such as an input function segmental decay, an inverse decay, an exponential decay, and an adaptive decay. The iterative training may be ended when iteration termination conditions are satisfied. The iteration termination conditions may include loss function convergence or being less than a preset threshold, rounds of iteration reaching a preset count of rounds, etc.

In some embodiments of the present disclosure, (1) more comprehensive coverage of image imaging may be facilitated to achieve by performing a short-axis scanning of the plurality of table positions; (2) the continuous input functions, continuous target lesion curves, imaging, etc., on the short-axis PET scanner may be achieved by using the information of the overlapping scanning area to obtain the continuous input functions, the target lesion curve, and the imaging over time; (3) differences in count rates of input functions, the count rates of the target lesion curves, and the count rates of the imaging between the adjacent table positions under different scanning conditions may be reduced by using the information of overlapping scanning areas.

Having thus descritable position the basic concepts, it may be rather apparent to those skilled in the art after reading this detailed disclosure that the foregoing detailed disclosure is intended to be presented by way of example only and is not limiting. Various alterations, improvements, and modifications may occur and are intended for those skilled in the art, though not expressly stated herein. These alterations, improvements, and modifications are intended to be suggested by the present disclosure, and are within the scope of the exemplary embodiments of the present disclosure.

Moreover, certain terminology has been used to describe embodiments of the present disclosure. For example, the terms "one embodiment," "an embodiment," and/or "some embodiments" mean that a particular feature, structure, or characteristic descritable position in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures, or characteristics may be combined as suitable position in one or more embodiments of the present disclosure.

Furthermore, the recited order of processing elements or sequences, or the use of numbers, letters, or other designations thereof, are not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the scope of the disclosed embodiments. For example, although the implementation of various components descritable position above may be embodied in a hardware device, it may also be implemented as a software-only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. This method of disclosure, however, is not to be interpreted as reflecting an intention that the claimed subject matter requires more features than are expressly recited in each claim. Rather, claimed subject matter may lie in less than all features of a single foregoing disclosed embodiment.

In some embodiments, the numbers expressing quantities or properties used to describe and claim certain embodiments of the application are to be understood as being modified in some instances by the term "about," "approximate," or "substantially." For example, "about," "approximate," or "substantially" may indicate ±20% variation of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the count of reported significant digits and by applying ordinary rounding techniques. Notwithstanding that the numerical ranges and parameters setting forth the broad scope of some embodiments of the application are approximations, the numerical values set forth in the specific examples are reported as precisely as practicable.

In closing, it is to be understood that the embodiments of the application disclosed herein are illustrative of the principles of the embodiments of the application. Other modifications that may be employed may be within the scope of the application. Therefore, by way of example, but not of limitation, alternative configurations of the embodiments of the application may be utilized in accordance with the teachings herein. Accordingly, embodiments of the present application are not limited to that precisely as shown and descritable position.

## Claims

1. A system (100), comprising:
at least one storage device (140) storing a set of instructions; and
at least one processor (120) in communication with the storage device, wherein when executing the set of instructions, the at least one processor is configured to cause the system to perform operations including:
obtaining a plurality of local input functions of an object, each of the plurality of local input functions being of a scanning area of the object, one of the plurality of local input functions being obtained based on a scan for one of a plurality of table positions where a table for placing the object is located, wherein adjacent table positions in the plurality of table positions correspond to an overlapping scanning area, wherein the obtaining a plurality of local input functions of an object includes:
obtaining scanning data collected by a Positron Emission Tomography (PET) scanner when the object is located on the table at the plurality of table positions; and
determining the plurality of local input functions based on the scanning data collected by the PET scanner, wherein a length of the object is greater than an axial FOV length of the PET scanner;
obtaining local input functions of at least one overlapping scanning area based on the plurality of local input functions; and
determining a continuous input function of at least a portion of a target scanning area based on the local input functions of the at least one overlapping scanning area.

2. The system of claim 1, wherein the obtaining a plurality of local input functions of an object includes:
for one table position of the plurality of table positions,
obtaining scanning data of the object acquired when the table is at the table position; and
determining the local input function of the scanning area corresponding to the table position based on the scanning data.

3. The system of claim 1, wherein the at least one overlapping scanning area includes a first overlapping scanning area between a table position and a previous table position and a second overlapping scanning area between the table position and a latter table position, and the determining a continuous input function of at least a portion of the target scanning area includes:
determining a ratio of a local input function of the first overlapping scanning area and a local input function of the second overlapping scanning area in a first time period for a scan of the table position;
determining at least one part of the continuous input function based on the ratio and a local input function of the second overlapping scanning area in a second time period for a scan of the latter table position; and
obtaining the continuous input function based on the at least one part of the continuous input function.

4. The system of claim 1, wherein the plurality of table positions include a target table position corresponding to a target scanning area, a first reference table position adjacent to and subsequent to the target table position, and a second reference table position adjacent to and subsequent to the first reference table position;
the at least one overlapping scanning area includes a first overlapping scanning area between the target table position and the first reference table position, and a second overlapping scanning area between the first reference table position and the second reference table position, and
determining a continuous input function of at least a portion of the target scanning area includes:
obtaining a first local input function of the first overlapping scanning area in the first time period;
obtaining a second local input function of the second overlapping scanning area in the first time period;
obtaining a third local input function of the second overlapping scanning area in the second time period;
obtaining a first ratio of the first local input function to the second local input function; and
obtaining at least a portion of the continuous input function in the second time period based on the first ratio and the third local input function of the second overlapping scanning area in the second time period.

5. The system of claim 4, wherein the obtaining a first ratio of the first local input function to the second local input function, includes:
obtaining an average value of ratios of the first local input function to the second local input function, at multiple time points in the first time period; and
determining the average value as the first ratio.

6. The system of claim 4, wherein the plurality of table positions further includes a third reference table position, the at least one overlapping scanning area further includes a third overlapping scanning area between the second reference table position and the third reference table position, and the determining a continuous input function of the at least portion of the target scanning area includes:
obtaining a fourth local input function of the third overlapping scanning area in the second time period;
obtaining a second ratio of the third local input function to the fourth local input function; and
obtaining the at least one a portion of the continuous input functions in a third time period based on a fifth local input function of the third overlapping scanning area in the third time period, the first ratio, and the second ratio.

7. The system of claim 6, wherein the obtaining a second ratio of the third local input function to the fourth local input function includes:
obtaining an average value of ratios of the third local input function to the fourth local input function at multiple time points in the second time period; and
determining the average value as the second ratio.

8. The system of claim 3, further including:
performing a plurality of rounds of scanning, each round of the plurality of rounds of scanning including scans of the plurality of table positions; and
determining the continuous input function of at least a portion of the target scanning area includes:
determining the continuous input function of the at least a portion of target scanning area in a round of scanning based on the local input functions of the at least one overlapping scanning area in the round of scanning.

9. A system (100), comprising:
at least one storage device (140) storing a set of instructions; and
at least one processor (120) in communication with the storage device, wherein when executing the set of instructions, the at least one processor is configured to cause the device to perform operations including:
obtaining a plurality of local parametric information of an object, each of the plurality of local parametric information being of a scanning area of the object, one of the plurality of local parametric information being obtained based on a scan for one of a plurality of table positions where a table for placing the object is located, wherein adjacent table positions in the plurality of table positions correspond to an overlapping scanning area, wherein the obtaining a plurality of local parametric information of an object includes:
obtaining scanning data collected by a Positron Emission Tomography (PET) scanner when the object is located on the table at the plurality of table positions; and
determining the plurality of local parametric information based on the scanning data collected by the PET scanner, wherein a length of the object is greater than an axial FOV length of the PET scanner;
obtaining overlapping parametric information of at least one overlapping scanning area based on the plurality of local parametric information; and determining continuous parametric information of at least a portion of a target scanning area based on the parametric information of the overlapping scanning area.

10. A method, comprising:
obtaining a plurality of local input functions of an object, each of the plurality of local input functions being of a scanning area of the object, one of the plurality of local input functions being obtained based on a scan for one of a plurality of table positions where a table for placing the object is located, wherein adjacent table positions in the plurality of table positions correspond to an overlapping scanning area, wherein the obtaining a plurality of local input functions of an object includes:
obtaining scanning data collected by a Positron Emission Tomography (PET) scanner when the object is located on the table at the plurality of table positions; and
determining the plurality of local input functions based on the scanning data collected by the PET scanner, wherein a length of the object is greater than an axial FOV length of the PET scanner;
obtaining local input functions of at least one overlapping scanning area based on the plurality of local input functions; and
determining a continuous input function of at least a portion of a target scanning area based on the local input functions of the overlapping scanning area.

11. The method of claim 10, wherein the at least one overlapping scanning area includes a first overlapping scanning area between a table position and a previous table position and a second overlapping scanning area between the table position and a latter table position, and the determining a continuous input function of the at least a portion of the target scanning area includes:
determining a ratio of a local input function of the first overlapping scanning area and a local input function of the second overlapping scanning area in a first time period for a scan of the table position;
determining at least one part of the continuous input function based on the ratio and a local input function of the second overlapping scanning area in a second time period for a scan of the latter table position; and
obtaining the continuous input function based on the at least one part of the continuous input function.

12. The method of claim 10, wherein the plurality of table positions include a target table position corresponding to a target scanning area, a first reference table position adjacent to and subsequent to the target table position, and a second reference table position adjacent to and subsequent to the first reference table position;
the at least one overlapping scanning area includes a first overlapping scanning area between the target table position and the first reference table position, and a second overlapping scanning area between the first reference table position and the second reference table position; and determining a continuous input function of at least a portion of the target scanning area includes:
obtaining a first local input function of the first overlapping scanning area in the first time period;
obtaining a second local input function of the second overlapping scanning area in the first time period;
obtaining a third local input function of the second overlapping scanning area in the second time period;
obtaining a first ratio of the first local input function to the second local input function; and
obtaining at least one part of the continuous input function in the second time period based on the first ratio and the third local input function.

13. The method of claim 12, wherein the obtaining a first ratio of the first local input function to the second local input function, including:
obtaining an average value of ratios of the first local input function to the second local input function at multiple time points in the first time period; and
determining the average value as the first ratio.

14. The method of claim 12, wherein the plurality of table positions further includes a third reference table position, the at least one overlapping scanning area further includes a third overlapping scanning area between the second reference table position and the third reference table position, and the determining a continuous input function of the at least portion of the target scanning area includes:
obtaining a fourth local input function of the third overlapping scanning area in the second time period;
obtaining a second ratio of the third local input function to the fourth local input function; and
obtaining the at least one part of the continuous input functions in a third time period based on a fifth local input function of the third overlapping scanning area in the third time period, the first ratio, and the second ratio.

## Patentansprüche

1. System (100), umfassend:
mindestens eine Speichervorrichtung (140), die einen Satz Anweisungen speichert; und
mindestens einen Prozessor (120), der in Kommunikation mit der Speichervorrichtung steht, wobei der mindestens eine Prozessor so konfiguriert ist, dass er, wenn er den Satz Anweisungen ausführt, das System dazu bringt, Operationen durchzuführen, die Folgendes beinhalten:
Erhalten mehrerer lokaler Eingabefunktionen eines Objekts, wobei jede der mehreren lokalen Eingabefunktionen einen Abtastbereich des Objekts betrifft, wobei eine der mehreren lokalen Eingabefunktionen basierend auf einer Abtastung für eine von mehreren Tischpositionen erhalten wird, an denen sich ein Tisch zur Platzierung des Objekts befindet, wobei benachbarte Tischpositionen in den mehreren Tischpositionen einem überlappenden Abtastbereich entsprechen, wobei das Erhalten mehrerer lokaler Eingabefunktionen eines Objekts Folgendes beinhaltet:
Erhalten von Scandaten, die von einem Positronen-Emissions-Tomographie-Scanner (PET-Scanner) gesammelt wurden, während sich das Objekt an einer der mehreren Tischpositionen auf dem Tisch befindet; und
Bestimmen der mehreren lokalen Eingangsfunktionen basierend auf den vom PET-Scanner gesammelten Scandaten, wobei eine Länge des Objekts größer ist als eine axiale FOV-Länge des PET-Scanners;
Erhalten lokaler Eingangsfunktionen mindestens eines überlappenden Abtastbereichs basierend auf den mehreren lokalen Eingangsfunktionen; und
Bestimmen einer kontinuierlichen Eingangsfunktion für mindestens einen Teil eines Zielabtastbereichs basierend auf den lokalen Eingangsfunktionen des mindestens einen überlappenden Abtastbereichs.

2. System nach Anspruch 1, wobei das Erhalten von mehreren lokalen Eingangsfunktionen eines Objekts Folgendes beinhaltet:
für eine Tischposition der mehreren Tischpositionen,
Erhalten von Scandaten des Objekts, die erfasst werden, während sich der Tisch in der Tischposition befindet; und
Bestimmen der lokalen Eingangsfunktion des Abtastbereichs, die der Tischposition entspricht, basierend auf den Scandaten.

3. System nach Anspruch 1, wobei der mindestens eine überlappende Abtastbereich einen ersten überlappenden Abtastbereich zwischen einer Tischposition und einer vorherigen Tischposition sowie einen zweiten überlappenden Abtastbereich zwischen der Tischposition und einer späteren Tischposition beinhaltet und das Bestimmen einer kontinuierlichen Eingangsfunktion mindestens eines Teils des Zielabtastbereichs Folgendes beinhaltet:
Bestimmen eines Verhältnisses einer lokalen Eingangsfunktion des ersten überlappenden Abtastbereichs und einer lokalen Eingangsfunktion des zweiten überlappenden Abtastbereichs in einem ersten Zeitraum für eine Abtastung der Tischposition;
Bestimmen mindestens eines Teils der kontinuierlichen Eingangsfunktion basierend auf dem Verhältnis und einer lokalen Eingangsfunktion des zweiten überlappenden Abtastbereichs in einem zweiten Zeitraum für eine Abtastung der letzteren Tischposition; und
Erhalten der kontinuierlichen Eingangsfunktion basierend auf mindestens einem Teil der kontinuierlichen Eingangsfunktion.

4. System nach Anspruch 1, wobei die mehreren Tischpositionen eine Zieltischposition, die einem Zielabtastbereich entspricht, eine erste Referenztischposition, die an die Zieltischposition angrenzt und auf diese folgt, und eine zweite Referenztischposition, die an die erste Referenztischposition angrenzt und auf diese folgt, beinhalten;
der mindestens eine überlappende Abtastbereich einen ersten überlappenden Abtastbereich zwischen der Zieltabellenposition und der ersten Referenztischposition sowie einen zweiten überlappenden Abtastbereich zwischen der ersten Referenztischposition und der zweiten Referenztischposition beinhaltet und
Bestimmen einer kontinuierlichen Eingangsfunktion für mindestens einen Teil des Zielabtastbereichs beinhaltet:
Erhalten einer ersten lokalen Eingangsfunktion des ersten überlappenden Abtastbereichs im ersten Zeitraum;
Erhalten einer zweiten lokalen Eingangsfunktion des zweiten überlappenden Abtastbereichs im ersten Zeitraum;
Erhalten einer dritten lokalen Eingangsfunktion des zweiten überlappenden Abtastbereichs im zweiten Zeitraum;
Erhalten eines ersten Verhältnisses der ersten lokalen Eingangsfunktion zur zweiten lokalen Eingangsfunktion; und
Erhalten mindestens eines Teils der kontinuierlichen Eingangsfunktion im zweiten Zeitraum basierend auf dem ersten Verhältnis und der dritten lokalen Eingangsfunktion des zweiten überlappenden Abtastbereichs im zweiten Zeitraum.

5. System nach Anspruch 4, wobei das Erhalten eines ersten Verhältnisses der ersten lokalen Eingangsfunktion zur zweiten lokalen Eingangsfunktion Folgendes beinhaltet:
Erhalten eines Mittelwerts von Verhältnissen der ersten lokalen Eingangsfunktion zur zweiten lokalen Eingangsfunktion zu mehreren Zeitpunkten im ersten Zeitraum; und
Bestimmen des Mittelwerts als das erste Verhältnis.

6. System nach Anspruch 4, wobei die mehreren Tischpositionen ferner eine dritte Referenztischposition beinhalten, der mindestens eine überlappende Abtastbereich ferner einen dritten überlappenden Abtastbereich zwischen der zweiten Referenztischposition und der dritten Referenztischposition beinhaltet und das Bestimmen einer kontinuierlichen Eingangsfunktion des mindestens einen Teils des Zielabtastbereichs Folgendes beinhaltet:
Erhalten einer vierten lokalen Eingangsfunktion des dritten überlappenden Abtastbereichs im zweiten Zeitraum;
Erhalten eines zweiten Verhältnisses der dritten lokalen Eingangsfunktion zur vierten lokalen Eingangsfunktion; und
Erhalten des mindestens einen Teils der kontinuierlichen Eingangsfunktionen in einem dritten Zeitraum basierend auf einer fünften lokalen Eingangsfunktion des dritten überlappenden Abtastbereichs im dritten Zeitraum, dem ersten Verhältnis und dem zweiten Verhältnis.

7. System nach Anspruch 6, wobei das Erhalten eines zweiten Verhältnisses der dritten lokalen Eingangsfunktion zur vierten lokalen Eingangsfunktion Folgendes beinhaltet:
Erhalten eines Mittelwerts von Verhältnissen der dritten lokalen Eingangsfunktion zur vierten lokalen Eingangsfunktion zu mehreren Zeitpunkten im zweiten Zeitraum; und
Bestimmen des Mittelwerts als das zweite Verhältnis.

8. System nach Anspruch 3, ferner beinhaltend:
Durchführen mehrerer Scanvorgänge, wobei jeder Vorgang der mehreren Scanvorgänge Abtastungen der mehreren Tischpositionen beinhaltet; und
Bestimmen der kontinuierlichen Eingangsfunktion mindestens eines Teils des Zielabtastbereichs beinhaltet:
Bestimmen der kontinuierlichen Eingangsfunktion des mindestens einen Teils des Zielabtastbereichs in einem Abtastvorgang basierend auf den lokalen Eingangsfunktionen des mindestens einen überlappenden Abtastbereichs in dem Abtastvorgang.

9. System (100), umfassend:
mindestens eine Speichervorrichtung (140), die einen Satz Anweisungen speichert; und
mindestens einen Prozessor (120), der in Kommunikation mit der Speichervorrichtung steht, wobei der mindestens eine Prozessor so konfiguriert ist, dass er, wenn er den Satz Anweisungen ausführt, die Vorrichtung dazu bringt, Operationen durchzuführen, die Folgendes beinhalten:
Erhalten von mehreren lokalen parametrischen Informationen eines Objekts, wobei jede der lokalen parametrischen Informationen einen Abtastbereich des Objekts betrifft, wobei eine der mehreren lokalen parametrischen Informationen basierend auf einer Abtastung für eine von mehreren Tischpositionen erhalten wird, an denen sich ein Tisch zur Platzierung des Objekts befindet, wobei benachbarte Tischpositionen in den mehreren Tischpositionen einem überlappenden Abtastbereich entsprechen, wobei das Erhalten mehrerer lokaler parametrischer Informationen eines Objekts beinhaltet:
Erhalten von Scandaten, die von einem Positronen-Emissions-Tomographie-Scanner (PET-Scanner) gesammelt wurden, während sich das Objekt an einer der mehreren Tischpositionen auf dem Tisch befindet; und
Bestimmen der mehreren lokalen parametrischen Informationen basierend auf den vom PET-Scanner gesammelten Scandaten, wobei eine Länge des Objekts größer ist als eine axiale FOV-Länge des PET-Scanners;
Erhalten überlappender parametrischer Informationen mindestens eines überlappenden Abtastbereichs basierend auf den mehreren lokalen parametrischen Informationen; und Bestimmen kontinuierlicher parametrischer Informationen mindestens eines Teils eines Zielabtastbereichs basierend auf den parametrischen Informationen des überlappenden Abtastbereichs.

10. Verfahren, umfassend:
Erhalten mehrerer lokaler Eingabefunktionen eines Objekts, wobei jede der mehreren lokalen Eingabefunktionen einen Abtastbereich des Objekts betrifft, wobei eine der mehreren lokalen Eingabefunktionen basierend auf einer Abtastung für eine von mehreren Tischpositionen erhalten wird, an denen sich ein Tisch zur Platzierung des Objekts befindet, wobei benachbarte Tischpositionen in den mehreren Tischpositionen einem überlappenden Abtastbereich entsprechen, wobei das Erhalten mehrerer lokaler Eingabefunktionen eines Objekts Folgendes beinhaltet:
Erhalten von Scandaten, die von einem Positronen-Emissions-Tomographie-Scanner (PET-Scanner) gesammelt wurden, während sich das Objekt an einer der mehreren Tischpositionen auf dem Tisch befindet; und
Bestimmen der mehreren lokalen Eingangsfunktionen basierend auf den vom PET-Scanner gesammelten Scandaten, wobei eine Länge des Objekts größer ist als eine axiale FOV-Länge des PET-Scanners;
Erhalten lokaler Eingangsfunktionen mindestens eines überlappenden Abtastbereichs basierend auf den mehreren lokalen Eingangsfunktionen; und
Bestimmen einer kontinuierlichen Eingangsfunktion für mindestens einen Teil eines Zielabtastbereichs basierend auf den lokalen Eingangsfunktionen des überlappenden Abtastbereichs.

11. Verfahren nach Anspruch 10, wobei der mindestens eine überlappende Abtastbereich einen ersten überlappenden Abtastbereich zwischen einer Tischposition und einer vorherigen Tischposition sowie einen zweiten überlappenden Abtastbereich zwischen der Tischposition und einer späteren Tischposition beinhaltet und das Bestimmen einer kontinuierlichen Eingangsfunktion des mindestens einen Teils des Zielabtastbereichs Folgendes beinhaltet:
Bestimmen eines Verhältnisses einer lokalen Eingangsfunktion des ersten überlappenden Abtastbereichs und einer lokalen Eingangsfunktion des zweiten überlappenden Abtastbereichs in einem ersten Zeitraum für eine Abtastung der Tischposition;
Bestimmen mindestens eines Teils der kontinuierlichen Eingangsfunktion basierend auf dem Verhältnis und einer lokalen Eingangsfunktion des zweiten überlappenden Abtastbereichs in einem zweiten Zeitraum für eine Abtastung der letzteren Tischposition; und
Erhalten der kontinuierlichen Eingangsfunktion basierend auf mindestens einem Teil der kontinuierlichen Eingangsfunktion.

12. Verfahren nach Anspruch 10, wobei die mehreren Tischpositionen eine Zieltischposition, die einem Zielabtastbereich entspricht, eine erste Referenztischposition, die an die Zieltischposition angrenzt und auf diese folgt, und eine zweite Referenztischposition, die an die erste Referenztischposition angrenzt und auf diese folgt, beinhalten;
der mindestens eine überlappende Abtastbereich einen ersten überlappenden Abtastbereich zwischen der Zieltischposition und der ersten Referenztischposition sowie einen zweiten überlappenden Abtastbereich zwischen der ersten Referenztischposition und der zweiten Referenztischposition beinhaltet; und das Bestimmen einer kontinuierlichen Eingangsfunktion für mindestens einen Teil des Zielabtastbereichs Folgendes beinhaltet:
Erhalten einer ersten lokalen Eingangsfunktion des ersten überlappenden Abtastbereichs im ersten Zeitraum;
Erhalten einer zweiten lokalen Eingangsfunktion des zweiten überlappenden Abtastbereichs im ersten Zeitraum;
Erhalten einer dritten lokalen Eingangsfunktion des zweiten überlappenden Abtastbereichs im zweiten Zeitraum;
Erhalten eines ersten Verhältnisses der ersten lokalen Eingangsfunktion zur zweiten lokalen Eingangsfunktion; und
Erhalten mindestens eines Teils der kontinuierlichen Eingangsfunktion im zweiten Zeitraum basierend auf dem ersten Verhältnis und der dritten lokalen Eingangsfunktion.

13. Verfahren nach Anspruch 12, wobei das Erhalten eines ersten Verhältnisses der ersten lokalen Eingangsfunktion zur zweiten lokalen Eingangsfunktion Folgendes beinhaltet:
Erhalten eines Mittelwerts von Verhältnissen der ersten lokalen Eingangsfunktion zur zweiten lokalen Eingangsfunktion zu mehreren Zeitpunkten im ersten Zeitraum; und
Bestimmen des Mittelwerts als das erste Verhältnis.

14. Verfahren nach Anspruch 12, wobei die mehreren Tischpositionen ferner eine dritte Referenztischposition beinhalten, der mindestens eine überlappende Abtastbereich ferner einen dritten überlappenden Abtastbereich zwischen der zweiten Referenztischposition und der dritten Referenztischposition beinhaltet und das Bestimmen einer kontinuierlichen Eingangsfunktion des mindestens einen Teils des Zielabtastbereichs Folgendes beinhaltet:
Erhalten einer vierten lokalen Eingangsfunktion des dritten überlappenden Abtastbereichs im zweiten Zeitraum;
Erhalten eines zweiten Verhältnisses der dritten lokalen Eingangsfunktion zur vierten lokalen Eingangsfunktion; und
Erhalten des mindestens einen Teils der kontinuierlichen Eingangsfunktionen in einem dritten Zeitraum basierend auf einer fünften lokalen Eingangsfunktion des dritten überlappenden Abtastbereichs im dritten Zeitraum, dem ersten Verhältnis und dem zweiten Verhältnis.

## Revendications

1. Système (100), comprenant :
au moins un dispositif de stockage (140) stockant un ensemble d'instructions ; et
au moins un processeur (120) en communication avec le dispositif de stockage, dans lequel, lors de l'exécution de l'ensemble d'instructions, le au moins un processeur est configuré pour amener le système à effectuer des opérations incluant :
l'obtention d'une pluralité de fonctions d'entrée locales d'un objet, chacune de la pluralité de fonctions d'entrée locales correspondant à une zone de balayage de l'objet, l'une de la pluralité de fonctions d'entrée locales étant obtenue sur la base d'un balayage pour l'une d'une pluralité de positions de table où se trouve une table destinée à accueillir l'objet, dans lequel des positions de table adjacentes dans la pluralité de positions de table correspondent à une zone de balayage à chevauchement, dans lequel l'obtention d'une pluralité de fonctions d'entrée locales d'un objet inclut :
l'obtention de données de balayage recueillies par un scanner à tomographie par émission de positons (PET) lorsque l'objet est situé sur la table au niveau de la pluralité de positions de table ; et
la détermination de la pluralité de fonctions d'entrée locales sur la base des données de balayage collectées par le scanner PET, dans lequel une longueur de l'objet est supérieure à une longueur de champ de vision axiale du scanner PET ;
l'obtention de fonctions d'entrée locales d'au moins une zone de balayage à chevauchement sur la base de la pluralité de fonctions d'entrée locales ; et
la détermination d'une fonction d'entrée continue d'au moins une portion d'une zone de balayage cible sur la base des fonctions d'entrée locales de la au moins une zone de balayage à chevauchement.

2. Système selon la revendication 1, dans lequel l'obtention d'une pluralité de fonctions d'entrée locales d'un objet inclut :
pour une position de table de la pluralité de positions de table,
l'obtention de données de balayage de l'objet acquises lorsque la table est dans la position de table ; et
la détermination de la fonction d'entrée locale de la zone de balayage correspondant à la position de table sur la base des données de balayage.

3. Système selon la revendication 1, dans lequel la au moins une zone de balayage à chevauchement inclut une première zone de balayage à chevauchement entre une position de table et une position de table précédente et une deuxième zone de balayage à chevauchement entre la position de table et une position de table ultérieure, et la détermination d'une fonction d'entrée continue d'au moins une portion de la zone de balayage cible inclut :
la détermination d'un rapport entre une fonction d'entrée locale de la première zone de balayage à chevauchement et une fonction d'entrée locale de la deuxième zone de balayage à chevauchement dans une première période de temps pour un balayage de la position de table ;
la détermination d'au moins une partie de la fonction d'entrée continue sur la base du rapport et d'une fonction d'entrée locale de la deuxième zone de balayage à chevauchement dans une deuxième période de temps pour un balayage de la position de table ultérieure ; et
l'obtention de la fonction d'entrée continue sur la base de la au moins une partie de la fonction d'entrée continue.

4. Système selon la revendication 1, dans lequel la pluralité de positions de table inclut une position de table cible correspondant à une zone de balayage cible, une première position de table de référence adjacente et ultérieure à la position de table cible, et une deuxième position de table de référence adjacente et ultérieure à la première position de table de référence ;
la au moins une zone de balayage à chevauchement inclut une première zone de balayage à chevauchement entre la position de table cible et la première position de table de référence, et une deuxième zone de balayage à chevauchement entre la première position de table de référence et la deuxième position de table de référence, et
la détermination d'une fonction d'entrée continue d'au moins une portion de la zone de balayage cible inclut :
l'obtention d'une première fonction d'entrée locale de la première zone de balayage à chevauchement dans la première période de temps ;
l'obtention d'une deuxième fonction d'entrée locale de la deuxième zone de balayage à chevauchement dans la première période de temps ;
l'obtention d'une troisième fonction d'entrée locale de la deuxième zone de balayage à chevauchement dans la deuxième période de temps ;
l'obtention d'un premier rapport entre la première fonction d'entrée locale et la deuxième fonction d'entrée locale ; et
l'obtention d'au moins une portion de la fonction d'entrée continue dans la deuxième période de temps sur la base du premier rapport et de la troisième fonction d'entrée locale de la deuxième zone de balayage à chevauchement dans la deuxième période de temps.

5. Système selon la revendication 4, dans lequel l'obtention d'un premier rapport entre la première fonction d'entrée locale et la deuxième fonction d'entrée locale inclut :
l'obtention d'une valeur moyenne de rapports entre la première fonction d'entrée locale et la deuxième fonction d'entrée locale, à de multiples moments de la première période de temps ; et
la détermination de la valeur moyenne comme premier rapport.

6. Système selon la revendication 4, dans lequel la pluralité de positions de table inclut en outre une troisième position de table de référence, la au moins une zone de balayage à chevauchement inclut en outre une troisième zone de balayage à chevauchement entre la deuxième position de table de référence et la troisième position de table de référence, et la détermination d'une fonction d'entrée continue de la au moins une portion de la zone de balayage cible inclut :
l'obtention d'une quatrième fonction d'entrée locale de la troisième zone de balayage à chevauchement dans la deuxième période de temps ;
l'obtention d'un second rapport entre la troisième fonction d'entrée locale et la quatrième fonction d'entrée locale ; et
l'obtention de la au moins une portion des fonctions d'entrée continues dans une troisième période de temps sur la base d'une cinquième fonction d'entrée locale de la troisième zone de balayage à chevauchement dans la troisième période de temps, du premier rapport et du second rapport.

7. Système selon la revendication 6, dans lequel l'obtention d'un second rapport entre la troisième fonction d'entrée locale et la quatrième fonction d'entrée locale inclut :
l'obtention d'une valeur moyenne de rapports entre la troisième fonction d'entrée locale et la quatrième fonction d'entrée locale à de multiples moments de la deuxième période de temps ; et
la détermination de la valeur moyenne comme second rapport.

8. Système selon la revendication 3, incluant en outre :
l'exécution d'une pluralité de cycles de balayage, chaque cycle de la pluralité de cycles de balayage incluant des balayages de la pluralité de positions de table ; et
la détermination de la fonction d'entrée continue d'au moins une portion de la zone de balayage cible inclut :
la détermination de la fonction d'entrée continue de la au moins une portion de zone de balayage cible dans un cycle de balayage sur la base des fonctions d'entrée locales de la au moins une zone de balayage à chevauchement dans le cycle de balayage.

9. Système (100), comprenant :
au moins un dispositif de stockage (140) stockant un ensemble d'instructions ; et
au moins un processeur (120) en communication avec le dispositif de stockage, dans lequel, lors de l'exécution de l'ensemble d'instructions, le au moins un processeur est configuré pour amener le dispositif à effectuer des opérations incluant :
l'obtention d'une pluralité d'informations paramétriques locales d'un objet, chacune de la pluralité d'informations paramétriques locales correspondant à une zone de balayage de l'objet, l'une de la pluralité d'informations paramétriques locales étant obtenue sur la base d'un balayage pour l'une d'une pluralité de positions de table où se trouve une table destinée à accueillir l'objet, dans lequel des positions de table adjacentes dans la pluralité de positions de table correspondent à une zone de balayage à chevauchement, dans lequel l'obtention d'une pluralité d'informations paramétriques locales d'un objet inclut :
l'obtention de données de balayage recueillies par un scanner à tomographie par émission de positons (PET) lorsque l'objet est situé sur la table au niveau de la pluralité de positions de table ; et
la détermination de la pluralité d'informations paramétriques locales sur la base des données de balayage collectées par le scanner PET, dans lequel une longueur de l'objet est supérieure à une longueur de champ de vision axiale du scanner PET ;
l'obtention d'informations paramétriques à chevauchement d'au moins une zone de balayage à chevauchement sur la base de la pluralité d'informations paramétriques locales ; et la détermination d'informations paramétriques continues d'au moins une portion d'une zone de balayage cible sur la base des informations paramétriques de la zone de balayage à chevauchement.

10. Procédé, comprenant :
l'obtention d'une pluralité de fonctions d'entrée locales d'un objet, chacune de la pluralité de fonctions d'entrée locales correspondant à une zone de balayage de l'objet, l'une de la pluralité de fonctions d'entrée locales étant obtenue sur la base d'un balayage pour l'une d'une pluralité de positions de table où se trouve une table destinée à accueillir l'objet, dans lequel des positions de table adjacentes dans la pluralité de positions de table correspondent à une zone de balayage à chevauchement, dans lequel l'obtention d'une pluralité de fonctions d'entrée locales d'un objet inclut :
l'obtention de données de balayage recueillies par un scanner à tomographie par émission de positons (PET) lorsque l'objet est situé sur la table au niveau de la pluralité de positions de table ; et
la détermination de la pluralité de fonctions d'entrée locales sur la base des données de balayage collectées par le scanner PET, dans lequel une longueur de l'objet est supérieure à une longueur de champ de vision axiale du scanner PET ;
l'obtention de fonctions d'entrée locales d'au moins une zone de balayage à chevauchement sur la base de la pluralité de fonctions d'entrée locales ; et
la détermination d'une fonction d'entrée continue d'au moins une portion d'une zone de balayage cible sur la base des fonctions d'entrée locales de la zone de balayage à chevauchement.

11. Procédé selon la revendication 10, dans lequel la au moins une zone de balayage à chevauchement inclut une première zone de balayage à chevauchement entre une position de table et une position de table précédente et une deuxième zone de balayage à chevauchement entre la position de table et une position de table ultérieure, et la détermination d'une fonction d'entrée continue de la au moins une portion de la zone de balayage cible inclut :
la détermination d'un rapport entre une fonction d'entrée locale de la première zone de balayage à chevauchement et une fonction d'entrée locale de la deuxième zone de balayage à chevauchement dans une première période de temps pour un balayage de la position de table ;
la détermination d'au moins une partie de la fonction d'entrée continue sur la base du rapport et d'une fonction d'entrée locale de la deuxième zone de balayage à chevauchement dans une deuxième période de temps pour un balayage de la position de table ultérieure ; et
l'obtention de la fonction d'entrée continue sur la base de la au moins une partie de la fonction d'entrée continue.

12. Procédé selon la revendication 10, dans lequel la pluralité de positions de table inclut une position de table cible correspondant à une zone de balayage cible, une première position de table de référence adjacente et ultérieure à la position de table cible, et une deuxième position de table de référence adjacente et ultérieure à la première position de table de référence ;
la au moins une zone de balayage à chevauchement inclut une première zone de balayage à chevauchement entre la position de table cible et la première position de table de référence et une deuxième zone de balayage à chevauchement entre la première position de table de référence et la deuxième position de table de référence ; et la détermination d'une fonction d'entrée continue d'au moins une portion de la zone de balayage cible inclut :
l'obtention d'une première fonction d'entrée locale de la première zone de balayage à chevauchement dans la première période de temps ;
l'obtention d'une deuxième fonction d'entrée locale de la deuxième zone de balayage à chevauchement dans la première période de temps ;
l'obtention d'une troisième fonction d'entrée locale de la deuxième zone de balayage à chevauchement dans la deuxième période de temps ;
l'obtention d'un premier rapport entre la première fonction d'entrée locale et la deuxième fonction d'entrée locale ; et
l'obtention d'au moins une partie de la fonction d'entrée continue dans la deuxième période de temps sur la base du premier rapport et de la troisième fonction d'entrée locale.

13. Procédé selon la revendication 12, dans lequel l'obtention d'un premier rapport entre la première fonction d'entrée locale et la deuxième fonction d'entrée locale inclut :
l'obtention d'une valeur moyenne de rapports entre la première fonction d'entrée locale et la deuxième fonction d'entrée locale à de multiples moments de la première période de temps ; et
la détermination de la valeur moyenne comme premier rapport.

14. Procédé selon la revendication 12, dans lequel la pluralité de positions de table inclut en outre une troisième position de table de référence, la au moins une zone de balayage à chevauchement inclut en outre une troisième zone de balayage à chevauchement entre la deuxième position de table de référence et la troisième position de table de référence, et la détermination d'une fonction d'entrée continue de la au moins une portion de la zone de balayage cible inclut :
l'obtention d'une quatrième fonction d'entrée locale de la troisième zone de balayage à chevauchement dans la deuxième période de temps ;
l'obtention d'un second rapport entre la troisième fonction d'entrée locale et la quatrième fonction d'entrée locale ; et
l'obtention de la au moins une partie des fonctions d'entrée continues dans une troisième période de temps sur la base d'une cinquième fonction d'entrée locale de la troisième zone de balayage à chevauchement dans la troisième période de temps, du premier rapport et du second rapport.
